# FASCICULE DE BREVET EUROPEEN

(11) **EP 1 637 190 B1**
(45) Date de publication et mention de la délivrance du brevet: **06.06.2007**
(21) Numéro de dépôt: 05290846.4
(22) Date de dépôt: 15.04.2005
(51) Int. Cl.: A61Q 9/02, A61K 8/02, A61K 8/73, A61K 8/81, A61K 8/92

(54) **Procédé de rasage avec un film anhydre; utilisation d'un film anhydre pour la préparation d'un produit de rasage; kits de rasage**
Verfahren zum Rasieren unter Verwendung eines wasserfreien Films; Verwendung eines wasserfreien Films zur Herstellung eines Rasierhilfsmittels; Rasierkit
Method for shaving using an anhydrous film; use of an anhydrous film for preparing a shaving product; shaving kits

(30) Priorité: 22.06.2004 FR 0451191
(43) Date de publication de la demande: 22.03.2006
(73) Titulaire: L'ORÉAL, 75008 Paris (FR)
(72) Inventeur: Legendre, Jean-Yves, 75015 Paris (FR)
(74) Mandataire: Miszputen, Laurent

(56) Documents cités:
- WO-A-02/05789
- WO-A-20/04032859
- FR-A- 2 331 980
- US-A- 4 963 351
- US-A1- 2002 187 112

## Description

La présente invention concerne un procédé de rasage consistant à partir d'un film anhydre soluble ou dispersible dans un solvant à générer une composition de rasage par mise en contact du film avec ledit solvant.

L'invention concerne également des kits de rasage contenant au moins un film anhydre soluble ou dispersible dans un solvant et au moins un rasoir notamment jetable et/ou un moyen d'étalement d'une composition de rasage.

Les produits utilisés par l'homme pour éliminer les poils de son corps à des fins esthétiques ou hygiéniques sont connus et variés, depuis le savon à raser en poudre, en barre ou en pain, des huiles jusqu'aux crèmes et mousses à raser et aux gels aqueux de rasage (se reporter à cet égard à l'article intitulé « Shaving Preparations - Origin of Shaving and Sales Trends », par Robert E. Sauté, publié dans l'ouvrage « The Chemistry and Manufacture of Cosmetics », Second Edition-1975-Vol IV-chapitre 64-pages 1313 à 1341, de Maison G. de Navarre).

A côté des gels de rasage traditionnels, on connaît également les gels transparents non-moussants et les gels de rasage à moussage différé (ou à effet de mousse différé) qui ont été plus précisément décrits dans de nombreux brevets tels que, par exemple, US 3 541 581, US 4 405 489, et FR 2 595 943. Ces gels à effet de mousse différé sont des compositions généralement conditionnées sous pression dans un dispositif aérosol qui délivre, sous l'effet d'un propulseur, des gels non moussants dans des conditions statiques, mais qui, sous l'action mécanique due à l'étalement du produit sur la peau, engendrent spontanément et presque instantanément une mousse sur cette dernière.

Les produits de rasage actuels sont généralement conditionnés sous forme de multi-doses tels que des pots, des flacons, des tubes ou des aérosols. Ces modes de conditionnement sont peu adaptés aux voyages car encombrants et pour certains d'entre eux fragiles. Des aérosols de faible volume ont été développés pour répondre à ce besoin mais leur fabrication est extrêmement coûteuse. Des articles de type lingette ou papier de rasage ont été proposés et notamment décrits dans les documents JP2000319147, JP09157143, JP2000256164 et US20011046513. Il s'agit de supports imprégnés d'une formulation fluide facilitant le rasage. Outre le fait que ces produits génèrent des problèmes de biodégradabilité néfastes pour l'environnement, ils sont très difficiles d'utilisation sur une peau non-rasée.

En effet, les produits de rasage doivent présenter une texture appropriée permettant un bon étalement sur la peau du visage, facilitant le déplacement du rasoir sur la surface de la peau à traiter et facilitant la coupe du poil. En outre, la formulation de rasage doit pouvoir très rapidement développer au contact de l'eau la texture recherchée de manière homogène et stable. Enfin, le produit de rasage doit être doté de bonnes qualités cosmétiques telles que l'hydratation, l'apport de douceur, le confort du rasage, l'absence d'effet collant et d'effet filant sur la peau. Ils doivent également facilement s'éliminer au rinçage à l'eau aussi bien de la peau que des lames de rasoir.

Il existe donc le besoin de disposer de nouvelles formes de produits de rasage particulièrement adaptés aux voyages, de faible volume, peu coûteux à la fabrication, faciles à mettre en oeuvre et qui présentent toutes les qualités d'un bon produit de rasage telles que mentionnées ci-dessus.

Ainsi, après de nombreuses recherches menées sur la question, la Demanderesse a maintenant découvert, de façon totalement inattendue et surprenante, qu'il était possible d'obtenir une formulation de rasage répondant à ce besoin à partir d'un film anhydre dispersible ou soluble dans un solvant en particulier une composition aqueuse. Un tel film anhydre est destiné à former extemporanément au contact dudit solvant une composition capable d'être appliquée sur la peau et de permettre un rasage efficace et confortable.

Cette découverte est à la base de la présente invention.

Certes, il est connu d'utiliser des films anhydres à dissolution immédiate au contact de l'eau ou de la salive, et des films comestibles visant à envelopper les aliments afin d'accroître leur durée de conservation, et cette technique a fait l'objet de nombreuses applications (Guilbert S. et al Technology and applications of edible protective films , Packaging Technology and Science , vol 8 pp 339-346, 1995 et de brevets comme par exemple US-A-5,965,708, US-A-5,962,053, JP-A-10/215792. De même, ce type de technologie est utilisé dans le domaine de la pharmacie pour administrer des principes actifs par voie orale sous forme de formulations à délitement buccal instantané par exemple dans les documents WO-A-2002/085119, WO-A-2002/043657, WO-A-2001/070194 . L'usage de ces films a également été étendu à l'administration de médicaments sur d'autres muqueuses, comme le vagin (EP-A-1,110,546) et sur les plaies (JP-A-63/220876).

En outre, le document JP-A-2002/212027 décrit l'obtention et la composition de préparations cosmétiques sous forme de films hydrosolubles sans revendiquer aucune utilisation particulière. Les documents WO-A-2002/05789, US-A-2002/0127254 et WO-A-2003/075812 décrivent l'obtention et l'administration de films polymériques anhydres pour une administration directe de compositions cosmétiques sur la peau préalablement mouillée. De plus, le document US-A-2003/0186826 décrit une composition cosmétique sèche à base de polymères et de tensioactifs, à administrer sur la peau ou les cheveux avec de l'eau. Toutefois, ces documents n'envisagent jamais la préparation extemporanée d'un produit destiné au rasage de la peau.

Il est connu dans la demande EP 1317916 d'utiliser en cosmétique des films hydratables à base d'amidon modifié et d'un actif notamment dans des compositions capillaires telles que des shampooings, des compositions de fixation des cheveux, des produits déodorants ou des produits de lavage. Toutefois, ce document n'envisage jamais la préparation extemporanée d'un produit destiné au rasage de la peau.

II est en outre connu dans la demande US2004/0029762 d'utiliser des feuilles hydratables de savon ou contenant une forte concentration de tensio-actifs destinées pour le nettoyage et l'hygiène de la peau. On connaît également dans la demande de brevet WO2004/032859 des feuilles hydrosolubles à base de polymère hydrosoluble, d'alcool polyvinylique et d'un tensioactif destinées pour le nettoyage et l'hygiène de la peau. Ces feuilles ne sont pas décrites pour une utilisation pour le rasage de la peau.

Ainsi l'objet principal de l'invention est un procédé de rasage de la peau caractérisé par le fait qu'il consiste :
(1) à partir d'un film anhydre soluble ou dispersible dans un solvant, à former extemporanément une composition de rasage par mise en contact du film avec ledit solvant puis
(2) à appliquer la composition ainsi formée sur la surface de la peau à raser et
(3) à raser les poils au moyen d'un rasoir.

Un autre objet de l'invention consiste en l'utilisation d'un film anhydre soluble ou dispersible dans un solvant et notamment une solution aqueuse pour la préparation d'une composition de rasage de la peau.

L'invention porte également sur un kit de rasage comprenant
(a) au moins un film anhydre soluble ou dispersible dans un solvant et notamment une composition aqueuse et
(b) au moins un rasoir notamment jetable et/ou
(c) un moyen d'étalement d'une composition de rasage comme par exemple un blaireau, une éponge ou une brosse.

Le solvant utilisé conformément au procédé de l'invention peut être un solvant organique comme par exemple une huile ou un mélange d'huiles couramment utilisées la fabrication des huiles de rasage ou bien une composition contenant au moins ledit solvant organique et éventuellement des actifs cosmétiques pour le confort et/ou le soin de la peau.

Le solvant utilisé conformément au procédé de l'invention sera de préférence une composition aqueuse et le film anhydre sera hydrosoluble ou dispersible dans l'eau. Ladite composition aqueuse peut être notamment de l'eau du robinet, une eau thermale, une eau de source, une eau parfumée, une composition aqueuse pouvant contenir des actifs cosmétiques pour le confort et/ou le soin de la peau.

Par "film", on entend dans la présente demande, un solide fin et préhensible. On entend par "fin", un solide ayant une épaisseur d'au maximum 5000 µm.

Le film peut être formé par une seule couche ou constitué de plusieurs couches superposées

Les films conformes à l'invention ont généralement une dimension adéquate pour pouvoir être facilement manipulé par l'utilisateur. Il peut avoir une forme de carré, de rectangle, de disque ou toute autre forme. Chaque film a de préférence une épaisseur de 50 µm à 5000 µm, de préférence de 100 à 2000 µm. Il peut avoir une surface de 1 à 50 cm² et de préférence de 2 à 30 cm².

Par ailleurs, par "film anhydre", on entend dans la présente demande, par contenant moins de 20 % en poids d'eau, de préférence moins de 10 % en poids par rapport au poids total du film,

On entend dans la présente demande par « film soluble », un film qui se dissout dans le solvant.

On entend dans la présente demande par « film dispersible », un film qui se disperse dans le solvant .

Selon un mode préférentiel de réalisation, les films anhydres conformes à la présente invention comprennent au moins un polymère hydrophile soluble dans l'eau et au moins des fibres hydrosolubles ou dispersibles dans l'eau.

A titre illustratif des polymères hydrophiles solubles dans l'eau utilisables selon l'invention, on peut notamment citer les polymères suivants :
- les polyvinylpyrrolidones ;
- les polymères cellulosiques, tels que l'hydroxyéthyl cellulose, l'hydroxypropyl cellulose, l'hydroxypropyl méthyl cellulose, la méthylcellulose, l'éthylhydroxyéthyl cellulose, la carboxyméthyl cellulose, et les dérivés quaternisés de la cellulose ; et
- les amidons éventuellement modifiés tels que ceux décrits dans la demande EP 1317916;
- les polymères d'origine naturelle éventuellement modifiés tels que la gomme arabique, la gomme de guar, les dérivés du xanthane, la gomme karaya, les glycoaminoglycanes, la gomme laque, la gomme sandaraque, les dammars, l'élémis, les copals, la pectine, la mannane et les galactomannanes, les glucomannanes et leurs dérivés
- les mélanges de ces polymères.

Le ou les polymères hydrosolubles est de préférence présent dans le film en une proportion allant de 5 à 80 % en poids et plus préférentiellement de 10 à 60% en poids par rapport au poids total du film.

Les fibres utilisables dans la composition de l'invention peuvent être choisies parmi les fibres d'origine naturelle notamment des fibres de cellulose extraites de végétaux et plus particulièrement les fibres de celluloses issues du bois, de la soie, du coton, de la laine, du lin, des fruits, de la betterave.

Parmi les fibres d'origine naturelle, on choisira plus particulièrement des fibres de cellulose de bois sous forme de pulpe. Parmi ces fibres, on peut citer notamment des produits correspondant par exemple aux références commerciales LIGNOCEL, LYOCELL, RAYON FLOCK, NATURAL RAYON, CELL-U-LASH, AV CELL, BIOFLUFF, TEMFILM, BIOFLOC.

Les fibres d'acide polylactique (PLA) issues du maïs peuvent également être utilisées.

Les fibres peuvent être introduites dans le film sous forme de papier dissolvable ou dispersible dans l'eau. De tels produits sont connus par exemple sous les noms commerciaux de DISSOLVO (Gilbreth) ou MDP (Mishima Paper)

Les fibres d'origine naturelle conformes à l'invention peuvent être mélangées à des résines ou des fibres synthétiques telles que polypropylène, polyéthylène, polyéthylène terephthalate, polyamide, ou polyvinyl acétate.

Parmi les fibres hydrosolubles, on peut citer tout particulièrement les fibres réalisées à partir d'alcool polyvinylique (PVA) comme par exemple les produits commercialisés par la société japonaise KURARAY sous la dénomination KURALON K II WN2. Le procédé de fabrication de ces fibres comporte l'emploi de solvants organiques. La section de ces fibres peut être sensiblement circulaire. La demande EP636716 décrit des fibres hydrosolubles à base de PVA et leur procédé de préparation.

Parmi les fibres hydrosolubles, on peut également citer les fibres d'oxyde de polyéthylène, les fibres d'acide polylactique telles que les fibres LACTRON de la société japonaise KANEBO, les fibres de polysaccharides commercialisées sous la dénomination LYSORB par la société LYSAC TECHNOLOGIES INC.

Les fibres sont de préférence présentes dans le film en une proportion allant de 1 à 90 % en poids et plus préférentiellement de 5 à 80 % en poids par rapport au poids total du film.

Selon un mode particulier de l'invention, les films anhydres peuvent comporter en plus au moins un tensioactif.

Les tensioactifs peuvent être choisis parmi les tensioactifs anioniques, amphotères, zwitterioniques ou non-ioniques et leurs mélanges.

Parmi les tensio-actifs anioniques utilisables selon l'invention, on peut citer les sels d'acides gras (ou savons) comportant une chaîne alkyle linéaire ou ramifiée, saturée ou insaturée, ayant de 6 à 30 atomes de carbone et de préférence 12 à 22 atomes de carbone.

Les bases susceptibles d'être utilisées pour neutraliser totalement ces acides gras peuvent être de préférence les bases minérales comme les hydroxydes de métal alcalins (soude et potasse), mais aussi les hydroxydes de métaux alcalino-terreux (de magnésium) ou l'ammoniaque ou encore les bases organiques comme les alcanolamines telles que la triéthanolamine, monéthanolamine, monoisopropanolamine ; les acides aminés comme la N-méthylglucamine, la lysine et l'arginine.

Comme exemples de sels d'acide gras, on utilisera plus particulièrement les sels d'alcanolamine (ie triethanolamine) ou les sels de potasse de l'acide laurique, l'acide myristique, l'acide palmitique ou l'acide stéarique ainsi que leurs mélanges.

Comme autres tensioactifs anioniques utilisables dans la présente invention, on peut notamment mentionner les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates ; les alkylsulfonates, les alkylamidesulfonates, les alkyl-arylsulfonates, les α-oléfine-sulfonates, les paraffine-sulfonates ; les alkylsulfosuccinates, les alkyléthersulfosuccinates, les alkylamide-sulfosuccinates ; les alkylsulfoacétates ; les acylsarcosinates ; et les acylglutamates, les groupes alkyle et acyle de tous ces composés comportant de 6 à 24 atomes de carbone et le groupe aryle désignant de préférence un groupe phényle ou benzyle. On peut également utiliser les esters d'alkyle en C₆-C₂₄ et d'acides polyglycoside-carboxyliques tels que les glucoside-citrates d'alkyle, les polyglycoside-tartrates d'alkyle, et les polyglycoside-sulfosuccinates d'alkyle ; les alkylsuifosuccinamates, les acyliséthionates et les N-acyltaurates, le groupe alkyle ou acyle de tous ces composés comportant de 12 à 20 atomes de carbone. Parmi les tensioactifs anioniques encore utilisables, on peut également citer les acyllactylates dont le groupe acyle comporte de 8 à 20 atomes de carbone et leurs mélanges.

En outre, on peut encore citer les acides d'alkyl-D-galactoside uroniques , les acides aikyl(C₆-C₂₄)éther-carboxyliques polyoxyalkylénés, les acides alkyl(C₆-C₂₄)aryl(C₆-C₂₄)éther-carboxyliques polyoxyalkylénés, les acides alkyl(C₆-C₂₄)amidoéther carboxyliques polyoxyalkylénés, en particulier ceux comportant de 2 à 50 groupements oxyde d'éthylène ; et leurs sels de métaux alcalins, d'ammonium, d'amines, d'aminoalcools ou de métaux alcalino-terreux.

Les agents tensioactifs amphotères, convenant dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique hydrosolubilisant tel que, par exemple, un groupe carboxylate, sulfonate, sulfate, phosphate ou phosphonate.

Parmi les dérivés d'amines, on peut citer les produits commercialisés sous la dénomination MIRANOL®, tels que décrits dans les brevets US-2 528 378 et US-2 781 354 et classés dans le dictionnaire CTFA, 3ème édition, 1982, sous les dénominations Amphocarboxy-glycinate et Amphocarboxypropionate.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique. A titre d'exemple, on peut citer le cocoamphodiacétate commercialisé sous la dénomination commerciale MIRANOL® C2M concentré par la société RHODIA.

Les agents tensioactifs amphotères, convenant dans la présente invention, peuvent être également choisis parmi les alkyl(C₈-C₂₀)bétaïnes, les sulfobétaïnes, les alkyl(C₈-C₂₀)amidoalkyl(C₆-C₈)-bétaïnes ou les alkyl(C₈-C₂₀)aMidoalkyl(C₆₋C₈)sulfobétaïnes ; et leurs mélanges et plus particulièrement les alkyl(C₈₋C₂₀)bétaïnes telles que la cocobétaine vendue sous les noms commerciaux MIRATAINE BB/FLA de RHODIA ou EMPIGEN BB/FL de Huntsman.

Les agents tensio-actifs non-ioniques peuvent être notamment choisis parmi les alcools, les alpha-diols, les alkyl(C₁-C₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitane éthoxylés ayant de 2 à 30 moles d'oxyde d'éthylène ; les esters d'acides gras du saccharose, les esters d'acides gras du polyéthylèneglycol, les alkyl(C₆₋C₂₄)polyglycosides, les dérivés de N-alkyl(C₆-C₂₄)glucamine, les oxydes d'amines tels que les oxydes d'alkyl(C₁₀-C₁₄)amines ou les oxydes de N-acyl(C₁₀-C₁₄)-aminopropylmorpholine ; et leurs mélanges.

Le ou les tensioactifs sont de préférence présents dans le film en une proportion allant de 5 à 80 % en poids et plus préférentiellement de 10 à 60 % en poids par rapport au poids total du film .

On choisira plus particulièrement les tensioactifs anioniques et plus particulièrement les sels d'acides gras (savons) tels que ceux définis ci.

Les films anhydres conformes à l'invention peuvent comporter en plus au moins un agent plastifiant.

Les agents plastifiants peuvent être choisis parmi les polyols tels que la glycérine, le polyéthylèneglycol, le dipropylèneglycol, le butylèneglycol, le pentylèneglycol ; les alcools de sucres comme le sorbitol, le manitol, le maltilol, le lactilol ; les mono-, di- ou oligosaccharides comme le glucose, le fructose, sucrose, maltose, lactose ; les acides polycarboxyliques comme l'acide citrique, l'acide maléique, l'acide succinique, l'acide polyacrylique ou polymaléique ; les polyesters comme la glycérintriacétate, le monoglycéride acétylé, diétylphthalate , tiéthylcitrate, tributylcitrate, acétyl triéthyl citrate acétyl tributyl citrate.

Les films anhydres conformes à l'invention peuvent encore contenir d'autres ingrédients ou actifs bien connus dans le domaine des produits de rasage.
On peut citer par exemple :
- des hydratants et parmi eux la glycérine et le sorbitol ;
- des agents apaisants tels que l'allantoine ou l'α-bisabolol ;
- des agents rafraîchissants et parfumants comme le menthol et ses dérivés,
- des lubrifiants tels que des silicones, des polydécènes, des polyvinylpyrrolidones ou des polymères cationiques,
- des agents surgraissants tels que les cires, les huiles végétales, animales ou minérales, des esters d'acides gras, des alcool gras ou des acides gras ;
- des émollients comme le polyéthylèneglycol, le polypropylèneglycol, le benzoate d'alcools en C₁₂/C₁₅ ou les stéarates de glycérol et de polyéthylèneglycol,
- des agents opacifiants,
- des agents stabilisants,
- des colorants,
- des parfums
- des conservateurs
- des antioxydants ;
- des agents régulateurs de pH
- des composés actifs

Parmi les composés actifs, on peut citer les vitamines notamment la vitamine E (tocophérol) et ses dérivés, la vitamine A (rétinol) et ses dérivés ; les acides gras polyinsaturés ;les huiles essentielles ; les alpha-hydroxyacides comme l'acide lactique ou glycolique, l'acide ascorbique (vitamine C) et ses dérivés ; l'urée, les aminoacides ; des oligopeptides ; les extraits végétaux notamment ceux de ginko bilboa ; les hydrolysats de peptides ou de protéines ;des oligo-éléments ; l'acide hyaluronique et ses sels ; le panthénol ; les béta-hydroxyacides notamment l'acide acétyl salicylique ou leurs mélanges.

Bien entendu, l'homme de l'art veillera à choisir le ou les éventuels composés complémentaires mentionnés ci-avant, de manière telle que les propriétés avantageuses attachées intrinsèquement aux films anhydres selon l'invention ne soient pas, ou substantiellement pas, altérées par la ou les adjonctions envisagées.
Les films anhydres selon l'invention peuvent être exempts de conservateur. Ceci est bien entendu avantageux en terme d'innocuité.

Avantageusement, les films anhydres conformes à l'invention sont utilisables à l'unité et présente une excellente conservation, y compris sans ajout de conservateur, en particulier grâce à sa nature sèche.

Les films anhydres de l'invention peuvent être conditionnés dans un article facilitant sa préhension, tel que celui décrit dans la demande de brevet FR0351002. Les films peuvent notamment êtres conditionnés dans une boîte plastique distributrice, dans un sachet individuel ou dans un blister. Les films anhydres hydratables selon l'invention peuvent être conditionnés dans un boîtier de type à tiroir ou à couvercle articulé sur un fond. Le boîtier peut comporter des moyens destinés à faciliter la distribution des articles. Les moyens de distribution peuvent être du type de ceux décrits par exemple dans les brevets US-A-2 973 882, GB-A-2 358 627, CH-A-461 025, ou US-A-6 578 732.

L'invention porte également sur un kit de rasage comprenant
(a) au moins un film anhydre soluble ou dispersible dans un solvant et notamment une composition aqueuse et
(b) au moins un rasoir notamment jetable et/ou
(c) un moyen d'étalement d'une composition de rasage comme par exemple un blaireau, une éponge ou une brosse.

Selon un mode particulier de l'invention, le kit de rasage peut comporter en plus le solvant destiné à être mélangé extemporanément avec le film du même kit et notamment la composition aqueuse

Ledit solvant et notamment la composition aqueuse peut se présenter sous une forme mono-dose, telle que des sachets, des tubes, des ampoules, des seringues pré-remplies, des capsules molles, des coques ou barquettes en plastique thermoformé. La solution aqueuse peut également se présenter sous une forme multi-doses classique comme un flacon et notamment être conditionnée dans un système distribuant une dose pré-définie. Un tel système peut être un flacon pompe, un aérosol, une pipette ou une seringue graduée, un compte-gouttes.
Selon un autre mode particulier de l'invention, le kit de rasage peut comporter également en plus une composition d'après-rasage.

Là encore, le produit après-rasage peut se présenter sous une forme mono-dose, telle que des sachets, des lingettes imprégnées, des tubes, des ampoules, des seringues pré-remplies, des capsules molles, des coques ou barquettes en plastique thermoformé. Le produit après-rasage peut également se présenter sous une forme multi-doses classique comme un flacon et notamment être conditionnée dans un système distribuant une dose pré-définie. Un tel système peut être un flacon pompe, un aérosol, une pipette ou une seringue graduée, un compte-gouttes.

Un exemple concret, mais nullement limitatif, illustrant l'invention va maintenant être donné.

### EXEMPLE 1 :

On a réalisé le film anhydre hydrosoluble de composition suivante
- Amidon modifié 30%en poids
- Pulpe de cellulose de bois 25% en poids
- Savon 35% en poids
- Glycérine 10% en poids

Après mise en contact du film avec de l'eau du robinet, on obtient instantanément un gel moussant dont la texture permet un étalement facile sur la peau du visage ainsi qu'un rasage efficace et confortable.

## Revendications

1. Procédé de rasage de la peau, **caractérisé par le fait qu**'il consiste :
(1) à partir d'un film anhydre soluble ou dispersible dans un solvant, à former extemporanément une composition de rasage par mise en contact du film avec ledit solvant ;
(2) à appliquer la composition ainsi formée sur la surface de la peau à raser et
(3) à raser les poils au moyen d'un rasoir.

2. Procédé selon la revendication 1, où le film anhydre a une épaisseur de 50 µm à 5000 µm, de préférence de 100 à 2000 µm.

3. Procédé selon la revendication 1 ou 2, où le film est formé par une seule couche ou constitué de plusieurs couches superposées.

4. Procédé selon l'une quelconque des revendications 1 à 3, où le film a une surface allant de 1 à 50 cm² et de préférence de 2 à 30 cm².

5. Procédé selon l'une quelconque des revendications 1 à 4, où le film anhydre est dispersible dans l'eau ou hydrosoluble et le solvant est une composition aqueuse.

6. Procédé selon l'une quelconque des revendications 1 à 5, où le film anhydre comprend au moins un polymère hydrophile soluble dans l'eau et des fibres hydrosolubles ou dispersibles dans l'eau.

7. Procédé selon la revendication 6, où le polymère hydrosoluble est choisi parmi
- les polyvinylpyrrolidones;
- les polymères cellulosiques;
- les amidons éventuellement modifiés ;
- les polymères d'origine naturelle éventuellement modifiés.

8. Procédé selon la revendication 6 ou 7, où le ou les polymères hydrosolubles sont présents dans le film en une proportion allant de 5 à 80 % en poids et plus préférentiellement de 10 à 60% en poids par rapport au poids total du film.

9. Procédé selon l'une quelconque des revendications 6 à 8, où les fibres comprennent au moins des fibres d'origine naturelle notamment des fibres de soie, de coton, de laine, de lin, des fibres de cellulose extraites de végétaux.

10. Procédé selon la revendication 9, où les fibres d'origine naturelle sont choisies parmi les fibres de celluloses issues du bois.

11. Procédé selon la revendication 9 ou 10, où les fibres d'origine naturelle sont mélangées à des résines ou des fibres synthétiques notamment polypropylène, polyéthylène, polyéthylène terephthalate, polyamide, ou polyvinyl acétate.

12. Procédé selon l'une quelconque des revendications 6 à 8, où les fibres sont des fibres hydrosolubles réalisées à partir de l'alcool polyvinylique (PVA).

13. Procédé selon l'une quelconque des revendications 6 à 12, où les fibres sont présentes dans le film en une proportion allant de 1 à 90 % en poids et plus préférentiellement de 5 à 80 % en poids par rapport au poids total du film.

14. Procédé selon l'une quelconque des revendications 1 à 13, où le film anhydre comporte en plus au moins un tensioactif.

15. Procédé selon la revendication 14, ou les tensioactifs sont choisis parmi les tensioactifs anioniques, amphotères, zwitterioniques ou non-ioniques et leurs mélanges.

16. Procédé selon la revendication 15, où les tensioactifs sont choisis parmi les tensioactifs anioniques .

17. Procédé selon la revendication 15 ou 16, où les tensioactifs sont choisis parmi les sels d'acides gras (savons) comportant une chaîne alkyle linéaire ou ramifiée, saturée ou insaturée, ayant de 6 à 30 atomes de carbone et de préférence 12 à 22 atomes de carbone.

18. Procédé selon la revendication 17, où les sels d'acides gras sont choisis parmi les sels d'alcanolamine ou les sels de potasse de l'acide laurique, l'acide myristique, l'acide palmitique ou de l'acide stéarique ainsi que leurs mélanges.

19. Procédé selon l'une quelconque des revendications 14 à 18, où le ou les tensioactifs sont présents dans le film en une proportion allant de 5 à 80 % en poids et plus préférentiellement de 10 à 60 % en poids par rapport au poids total du film.

20. Procédé selon l'une quelconque des revendications 1 à 19, où le film anhydre comporte en plus au moins un agent plastifiant.

21. Procédé selon l'une quelconque des revendications 1 à 20, où le film anhydre comporte en plus au moins un additif choisi parmi des hydratants ; des agents apaisants ; des agents rafraîchissants et parfumants; des lubrifiants ; des agents surgraissants ; des émollients; des agents opacifiants ; des agents stabilisants ; des colorants; des parfums; des conservateurs ; des antioxydants; des agents régulateurs de pH; des composés actifs.

22. Procédé selon la revendication 21, où les composés actifs sont choisis parmi la vitamine E (tocophérol) et ses dérivés, la vitamine A (rétinol) et ses dérivés les acides gras polyinsaturés les huiles essentielles ; les alpha-hydroxyacides, notamment l'acide lactique ou glycolique, l'acide ascorbique (vitamine C) et ses dérivés ; l'urée : les aminoacides ; des oligopeptides; les extraits végétaux notamment ceux de ginko bilboa ; les hydrolysats de peptides ou de protéines ;des oligo-éléments ; l'acide hyaluronique et ses sels ; le panthénol ; les béta-hydroxyacides notamment l'acide acétyl salicylique ou leurs mélanges.

23. Utilisation d'un film anhydre tel que défini dans l'une des revendications précédentes pour la préparation d'une composition de rasage de la peau.

24. Kit de rasage **caractérisé par le fait qu'**il comprend
a) au moins un film anhydre tel que défini dans l'une des revendications 1 à 22 et
(b) au moins un rasoir notamment jetable et/ou
(c) un moyen d'étalement d'une composition de rasage.

25. Kit de rasage selon la revendication 24 comprenant en plus le solvant destiné à être mélangé extemporanément au film anhydre.

26. Kit de rasage selon la revendication 24 ou 25, comprenant en plus une composition d'après-rasage.

## Claims

1. Method for shaving the skin, **characterized in that** it consists:
(1) starting from an anhydrous film which is soluble or dispersible in a solvent, in forming at the time of use a shaving composition by bringing the film into contact with the said solvent,
(2) in applying the composition thus formed to the surface of the skin to be shaved, and
(3) in shaving the hairs using a razor.

2. Method according to Claim 1, where the anhydrous film has a thickness of 50 µm to 5000 µm, preferably of 100 to 2000 µm.

3. Method according to Claim 1 or 2, where the film is formed by a single layer or is composed of several superimposed layers.

4. Method according to any one of Claims 1 to 3, where the film has a surface area ranging from 1 to 50 cm² and preferably from 2 to 30 cm².

5. Method according to any one of Claims 1 to 4, where the anhydrous film is dispersible or soluble in water and the solvent is an aqueous composition.

6. Method according to any one of Claims 1 to 5, where the anhydrous film comprises at least one hydrophilic polymer which is soluble in water and fibres which are soluble or dispersible in water.

7. Method according to Claim 6, where the water-soluble polymer is chosen from:
- polyvinylpyrrolidones;
- cellulose polymers;
- optionally modified starches;
- optionally modified polymers of natural origin.

8. Method according to Claim 6 or 7, where the water-soluble polymer or polymers are present in the film in a proportion ranging from 5 to 80% by weight and more preferably from 10 to 60% by weight, with respect to the total weight of the film.

9. Method according to any one of Claims 6 to 8, where the fibres comprise at least fibres of natural origin, in particular fibres of silk, cotton, wool or flax or cellulose fibres extracted from plants.

10. Method according to Claim 9, where the fibres of natural origin are chosen from cellulose fibres resulting from wood.

11. Method according to Claim 9 or 10, where the fibres of natural origin are mixed with synthetic resins or fibres, in particular polypropylene, polyethylene, poly(ethylene terephthalate), polyamide or poly(vinyl acetate) resins or fibres.

12. Method according to any one of Claims 6 to 8, where the fibres are water-soluble fibres produced from poly(vinyl alcohol) (PVA).

13. Method according to any one of Claims 6 to 12, where the fibres are present in the film in a proportion ranging from 1 to 90% by weight and more preferably from 5 to 80% by weight, with respect to the total weight of the film.

14. Method according to any one of Claims 1 to 13, where the anhydrous film additionally comprises at least one surfactant.

15. Method according to Claim 14, where the surfactants are chosen from anionic, amphoteric, zwitterionic or nonionic surfactants and their mixtures.

16. Method according to Claim 15, where the surfactants are chosen from anionic surfactants.

17. Method according to Claim 15 or 16, where the surfactants are chosen from salts of fatty acids (soaps) comprising a saturated or unsaturated, linear or branched, alkyl chain having from 6 to 30 carbon atoms and preferably 12 to 22 carbon atoms.

18. Method according to Claim 17, where the salts of fatty acids are chosen from alkanolamine salts or potassium hydroxide salts of lauric acid, myristic acid, palmitic acid or stearic acid, and their mixtures.

19. Method according to any one of Claims 14 to 18, where the surfactant or surfactants are present in the film in a proportion ranging from 5 to 80% by weight and more preferably from 10 to 60% by weight, with respect to the total weight of the film.

20. Method according to any one of Claims 1 to 19, where the anhydrous film additionally comprises at least one plasticizing agent.

21. Method according to any one of Claims 1 to 20, where the anhydrous film additionally comprises at least one additive chosen from moisturizing agents; soothing agents; refreshing and scenting agents; lubricants; superfatting agents; emollients; opacifying agents; stabilizing agents; colorants; fragrances; preservatives; antioxidants; pH-regulating agents; or active compounds.

22. Method according to Claim 21, where the active compounds are chosen from vitamin E (tocopherol) and its derivatives or vitamin A (retinol) and its derivatives; polyunsaturated fatty acids; essential oils; α-hydroxy acids, in particular lactic or glycolic acid, or ascorbic acid (vitamin C) and their derivatives; urea; amino acids; oligopeptides; plant extracts, in particular those of ginkgo biloba; peptide or protein hydrolysates; trace elements; hyaluronic acid and its salts; panthenol; β-hydroxy acids, in particular acetylsalicylic acid; or their mixtures.

23. Use of an anhydrous film as defined in one of the preceding claims in the preparation of a composition for shaving the skin.

24. Shaving kit, **characterized in that** it comprises:
(a) at least one anhydrous film as defined in one of Claims 1 to 22 and
(b) at least one razor, in particular a disposable razor, and/or
(c) a means for spreading a shaving composition.

25. Shaving kit according to Claim 24, additionally comprising the solvent intended to be mixed at the time of use with the anhydrous film,

26. Shaving kit according to Claim 24 or 25, additionally comprising an aftershave composition.

## Patentansprüche

1. Verfahren zum Rasieren der Haut, **dadurch gekennzeichnet, dass** es darin besteht,
(1) aus einem wasserfreien Film, der in einem Lösemittel dispergierbar oder löslich ist, bedarfsgemäß eine Rasierzusammensetzung zu erzeugen, indem der Film mit dem Lösemittel in Kontakt gebracht wird;
(2) eine so erzeugte Zusammensetzung auf die Oberfläche der zu rasierenden Haut aufzutragen und
(3) die Körperhaare mit einem Rasierer zu rasieren.

2. Verfahren nach Anspruch 1, wobei der wasserfreie Film eine Dicke von 50 bis 5000 µm und vorzugsweise 100 bis 2000 µm aufweist.

3. Verfahren nach Anspruch 1 oder 2, wobei der Film aus einer einzigen Schicht gebildet ist oder aus mehreren übereinander angeordneten Schicht besteht.

4. Verfahren nach einem der Ansprüche 1 bis 3, wobei der Film eine Oberfläche hat, die im Bereich von 1 bis 50 cm² und vorzugsweise 2 bis 30 cm² liegt.

5. Verfahren nach einem der Ansprüche 1 bis 4, wobei der wasserfreie Film in Wasser dispergierbar oder wasserlöslich ist und das Lösemittel eine wässrige Zusammensetzung ist.

6. Verfahren nach einem der Ansprüche 1 bis 5, wobei der wasserfreie Film mindestens ein hydrophiles Polymer, das in Wasser löslich ist, und wasserlösliche oder in Wasser dispergierbare Fasern umfasst.

7. Verfahren nach Anspruch 6, wobei das wasserlösliche Polymer ausgewählt wird unter:
- den Polyvinylpyrrolidonen;
- den Cellulosepolymeren;
- den gegebenenfalls modifizierten Stärken;
- den gegebenenfalls modifizierten Polymeren natürlicher Herkunft.

8. Verfahren nach Anspruch 6 oder 7, wobei das oder die wasserlöslichen Polymere in dem Film in einem Anteil enthalten sind, der im Bereich von 5 bis 80 Gew.-% und noch bevorzugter 10 bis 60 Gew.-%, bezogen auf das Gesamtgewicht des Films, liegt.

9. Verfahren nach einem der Ansprüche 6 bis 8, wobei die Fasern mindestens Fasern natürlicher Herkunft umfassen, insbesondere Seidenfasern, Baumwollfasern, Wollfasern, Flachsfasern, aus Pflanzen gewonnene Cellulosefasern.

10. Verfahren nach Anspruch 9, wobei die Fasern natürlicher Herkunft unter den Cellulosefasern ausgewählt werden, die von Holz stammen.

11. Verfahren nach Anspruch 9 oder 10, wobei die Fasern natürlicher Herkunft mit Syntheseharzen oder Synthesefasern, insbesondere Polypropylen, Polyethylen, Polyethylenterephthalat, Polyamid oder Polyvinylacetat, vermischt werden.

12. Verfahren nach einem der Ansprüche 6 bis 8, wobei die Fasern wasserlösliche Fasern sind, die aus Polyvinylalkohol (PVA) erzeugt werden.

13. Verfahren nach einem der Ansprüche 6 bis 12, wobei die Fasern in dem Film in einem Anteil enthalten sind, der im Bereich von 1 bis 90 Gew.-% und noch bevorzugter 5 bis 80 Gew.-%, bezogen auf das Gesamtgewicht des Films, liegt.

14. Verfahren nach einem der Ansprüche 1 bis 13, wobei der wasserfreie Film außerdem mindestens einen grenzflächenaktiven Stoff enthält.

15. Verfahren nach Anspruch 14, wobei die grenzflächenaktiven Stoffe unter den anionischen, den amphoteren, den zwitterionischen und den nichtionischen grenzflächenaktiven Stoffen und deren Gemischen ausgewählt werden.

16. Verfahren nach Anspruche 15, wobei die grenzflächenaktiven Stoffe unter den anionischen grenzflächenaktiven Stoffen ausgewählt werden.

17. Verfahren nach Anspruch 15 oder 16, wobei die grenzflächenaktiven Stoffe unter den Salzen von Fettsäuren (Seifen) ausgewählt werden, die eine geradkettige oder verzweigte, gesättigte oder ungesättigte Alkylkette aufweisen, die 6 bis 30 Kohlenstoffatome und vorzugsweise 12 bis 22 Kohlenstoffatome hat.

18. Verfahren nach Anspruch 17, wobei die Salze der Fettsäuren unter den Alkanolaminsalzen und den Kaliumsalzen von Laurinsäure, Myristinsäure, Palmitinsäure und Stearinsäure sowie deren Gemischen ausgewählt werden.

19. Verfahren nach einem der Ansprüche 14 bis 18, wobei der oder die grenzflächenaktiven Stoffe in dem Film in einem Anteil enthalten sind, der im Bereich von 5 bis 80 Gew.-% und noch bevorzugter 10 bis 60 Gew.-%, bezogen auf das Gesamtgewicht des Films, liegt.

20. Verfahren nach einem der Ansprüche 1 bis 19, wobei der wasserfreie Film außerdem mindestens einen Weichmacher enthält.

21. Verfahren nach einem der Ansprüche 1 bis 20, wobei der wasserfreie Film außerdem mindestens einen Zusatzstoff enthält, der unter den Hydratisierungsmitteln, den reizlindernden Mitteln, den erfrischenden und parfümierenden Mitteln, den Gleitmitteln, den rückfettenden Mitteln, den Emollientien, den Trübungsmitteln, den Stabilisierungsmitteln, den Farbmitteln, den Parfüms, den Konservierungsmitteln, den Antioxidantien, den den pH-Wert regulierenden Mitteln, den Wirkstoffen ausgewählt wird.

22. Verfahren nach Anspruch 21, wobei die Wirkstoffe unter Vitamin E (Tocopherol) und seinen Derivaten, Vitamin A (Retinol) und seinen Derivaten; mehrfach ungesättigten Fettsäuren, etherischen Ölen, α-Hydroxysäuren, insbesondere Milchsäure oder Glycolsäure, Ascorbinsäure (Vitamin C) und ihren Derivaten, Harnstoff, Aminosäuren, Oligopeptiden, pflanzlichen Extrakten, insbesondere Extrakten von Gingko biloba, Hydrolysaten von Peptiden oder Proteinen, Spurenelementen, Hyaluronsäure und ihren Salzen, Panthenol, β-Hydroxysäuren, insbesondere Acetylsalicylsäure, und deren Gemischen ausgewählt werden.

23. Verwendung eines wie in einem der vorhergehenden Ansprüche definierten wasserfreien Films für die Herstellung einer Zusammensetzung zum Rasieren der Haut.

24. Rasierkit, **dadurch gekennzeichnet, dass** er umfasst:
(a) mindestens einen wie in einem der Ansprüche 1 bis 22 definierten wasserfreien Film und
(b) mindestens einen Rasierer, insbesondere Einwegrasierer, und/oder
(c) eine Einrichtung zum Verteilen einer Rasierzusammensetzung.

25. Rasierkit nach Anspruch 24, der außerdem das Lösemittel umfasst, das dafür vorgesehen ist, bedarfsgemäß mit dem wasserfreien Film vermischt zu werden.

26. Rasierkit nach Anspruch 24 oder 25, der außerdem eine AfterShave-Zusammensetzung umfasst.
